# FASCICULE DE BREVET EUROPEEN

(11) **EP 3 003 494 B1**
(45) Date de publication et mention de la délivrance du brevet: **01.08.2018**
(21) Numéro de dépôt: 14729623.0
(22) Date de dépôt: 28.05.2014
(51) Int. Cl.: A61Q 5/02, A61K 8/60, A61K 8/87

(54) **COMPOSITION COSMÉTIQUE COMPRENANT DES POLYMÈRES ASSOCIATIFS NON IONIQUES ET DES TENSIOACTIFS NON IONIQUES, ET PROCÉDÉ DE TRAITEMENT COSMÉTIQUE**
KOSMETISCHE ZUSAMMENSETZUNG MIT NICHTIONISCHEN ASSOZIATIVEN POLYMEREN UND NICHTIONISCHEN TENSIDEN UND VERFAHREN ZUR KOSMETISCHEN BEHANDLUNG
COSMETIC COMPOSITION CONTAINING NON-IONIC ASSOCIATIVE POLYMERS AND NON-IONIC SURFACTANTS, AND METHOD FOR COSMETIC TREATMENT

(30) Priorité: 03.06.2013 FR 1355035
(43) Date de publication de la demande: 13.04.2016
(73) Titulaire: L'Oréal, 75008 Paris (FR)
(72) Inventeur: D'ARRAS, Marie-Florence, 92110 Clichy (FR); MATHONNEAU, Estelle, 93400 Saint-Ouen (FR)
(74) Mandataire: Dodin, Catherine
(86) Numéro de dépôt international: PCT/EP2014/061033
(87) Numéro de publication internationale: WO 2014/195198

(56) Documents cités:
- EP-A1- 0 555 155
- EP-A1- 1 550 435
- FR-A1- 2 935 267

## Description

La présente invention concerne une composition cosmétique, selon la revendication 1 ainsi qu'un procédé de traitement cosmétique mettant en oeuvre ladite composition.

Les chevelures ont tendance à perdre certaines de leurs qualités sous l'action de facteurs tels que le regraissage naturel, la sueur, l'élimination de squames, la pollution ou l'humidité notamment. L'aspect visuel ainsi que le toucher des cheveux peut ainsi être dégradé. Le regraissage, par exemple, alourdit les cheveux qui ont alors tendance à se mettre en paquets. Les cheveux peuvent être plus difficiles à coiffer, et avoir une brillance grasse ou un toucher ciré désagréable.
Il est connu de nettoyer les cheveux avec des shampooings qui sont généralement des compositions aqueuses contenant de grandes quantités de tensioactifs, qui sont généralement des tensioactifs anioniques, seuls ou en associations avec des tensioactifs amphotères et/ou non ioniques. Les quantités totales de tensioactifs mises en oeuvre dépassent le plus souvent 10% en poids de matière active, par rapport au poids total de la composition cosmétique.
Ces shampooings à base de quantités importantes de tensioactifs anioniques peuvent générer des désagréments tels que des picotements du cuir chevelu ou bien des yeux, lorsqu'ils sont mis en contact avec le shampoing.
Par ailleurs, ces tensioactifs peuvent altérer, au fur et à mesure des applications, les propriétés cosmétiques des cheveux ce qui conduit à la nécessité d'utiliser également des agents conditionneurs comme des polymères cationiques, des silicones ou des huiles non siliconées.
En outre, le rinçage des compositions cosmétiques à forte teneur en tensioactifs peut être souvent long. Enfin, pour éviter les coulures à l'application et notamment les coulures dans les yeux, les shampooings doivent généralement être épaissis; mais leur épaississement peut poser des problèmes de stabilité de la composition.
Afin de pallier ces différents problèmes, il a été proposé, par exemple par la demande FR2935267, d'ajouter des polymères associatifs aux compositions de shampoing, ce qui permettait de diminuer leur teneur en tensioactifs classiques. A partir d'une certaine concentration, ces polymères associatifs ont un pouvoir détergent suffisant pour permettre le nettoyage des cheveux en présence de très faibles quantités de tensioactifs voire même en l'absence de ces tensioactifs.
Toutefois, les compositions ainsi obtenues, même si elles permettent une détergence similaire à celle obtenue avec un shampoing classique, présentent encore un caractère moussant insuffisant; en outre, les propriétés cosmétiques conférées aux cheveux ne sont pas encore totalement satisfaisantes, en particulier sur cheveux secs.

La présente invention a pour but de proposer des compositions cosmétiques capillaires palliant ces inconvénients, et notamment susceptibles de générer une mousse adéquate, en qualité comme en quantité, et apportant aux cheveux, des propriétés cosmétiques satisfaisantes, tout particulièrement sur cheveux secs.

L'invention a donc pour objet une composition cosmétique, selon la revendication 1. La composition selon l'invention est non colorante. Par composition non colorante, on entend selon la présente invention, une composition ne contenant pas de colorant des fibres kératiniques tels que les colorants directs ou les précurseurs de colorant d'oxydation (bases et/ou coupleurs). S'ils sont présents, leur teneur ne dépasse pas 0,005% en poids par rapport au poids total de la composition. En effet, à une telle teneur, seule la composition serait teintée, c'est-à-dire qu'on n'observerait pas d'effet de coloration des fibres kératiniques.
Dans la présente description, l'expression "au moins un" est équivalente à l'expression "un ou plusieurs" et peut y être substituée.
Dans la présente description, l'expression "compris entre" est équivalente à l'expression "allant de" et peut y être substituée; dans ces expressions, les bornes sont considérées comme incluses.

### Polymère non ionique associatif

La composition selon l'invention comprend donc un ou plusieurs polymères non ioniques associatifs.
Au sens de la présente invention, on entend par "polymère" tout composé issu de la polymérisation par polycondensation ou de la polymérisation radicalaire de monomères dont l'un au moins est différent d'un oxyde d'alkylène et d'un composé monofonctionnel de formule RX, R désignant un groupe alkyle ou alkényle en C10-C30, éventuellement hydroxylé, et X désignant un groupement acide carboxylique, amine, amide, hydroxyle, ester. Sont en particulier exclus tous les composés issus uniquement de la simple condensation d'un oxyde d'alkylène sur un alcool gras, un ester gras, un acide gras, un amide gras, une amine grasse.
Au sens de la présente invention, on entend par "polymère associatif" un polymère amphiphile capable, dans un milieu aqueux, de s'associer réversiblement avec lui-même ou avec d'autres molécules. Il comporte généralement dans sa structure chimique au moins une zone ou groupement, hydrophile et au moins une zone ou groupement, hydrophobe.
Par "groupement hydrophobe", on entend un radical ou un polymère à chaîne hydrocarbonée, saturée ou non, linéaire ou ramifiée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et préférentiellement de 18 à 30 atomes de carbone. Préférentiellement, le groupement hydrophobe hydrocarboné provient d'un composé monofonctionnel. A titre d'exemple, le groupement hydrophobe peut être issu d'un alcool gras tel que l'alcool stéarylique, l'alcool dodécylique, l'alcool décylique ou bien d'un alcool gras polyalkyléné comme le stéareth-100. Il peut également désigner un polymère hydrocarboné tel que par exemple le polybutadiène.

Le ou les polymères associatifs non ioniques sont choisis parmi les polyuréthanes polyéthers. Les polyuréthanes polyéthers non ioniques associatifs selon l'invention sont des copolymères triblocs dont la séquence hydrophile est une chaîne polyoxyéthylénée comportant de 50 à 1000, notamment de 100 à 300, groupements oxyéthylénés; et comportant au moins deux chaînes lipophiles hydrocarbonées ayant de 6 à 30 atomes de carbone, séparées par ladite séquence hydrophile, lesdites chaînes lipophiles hydrocarbonées pouvant être des chaînes pendantes ou des chaînes en bout de séquence hydrophile.

De préférence, on utilise un polyuréthane polyéther non ionique associatif susceptible d'être obtenu par polycondensation d'au moins trois composés comprenant (i) au moins un polyéthylèneglycol comprenant de 100 à 180 moles d'oxyde d'éthylène, (ii) un alcool stéarylique polyoxyéthyléné comprenant 100 moles d'oxyde d'éthylène et (iii) un diisocyanate.
Un tel polymère est notamment proposé par la société ELEMENTIS sous l'appellation RHEOLATE FX 1100 ® qui est un polycondensat de polyéthylèneglycol à 136 moles d'oxyde d'éthylène, d'alcool stéarylique polyoxyéthyléné à 100 moles d'oxyde d'éthylène et de hexaméthylène diisocyanate (HDI) ayant poids moléculaire moyen en poids (Mw) de 30000 (nom INCI: PEG-136/STEARETH-100/HDI COPOLYMER).
Selon une autre préférence, on utilise un polyuréthane polyéther non ionique associatif susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.
De tels polymères sont notamment proposés par la société ROHM & HAAS sous les appellations Aculyn 46 ® et Aculyn 44 ® .
L'Aculyn 46 ® est un polycondensat de polyéthylèneglycol à 150 ou 180 moles d'oxyde d'éthylène, d'alcool stéarylique et de méthylène bis(4-cyclohexyl-isocyanate) (SMDI) à 15% en poids dans une matrice de maltodextrine (4%) et d'eau (81%) (nom INCI : PEG-150/STEARYL ALCOHOL/SMDI COPOLYMER).
L'Aculyn 44 ® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%) (nom INCI : PEG-150/DECYL ALCOHOL/SMDI COPOLYMER).

La composition selon l'invention comprend les polymères non ioniques associatifs en une quantité allant de 2,5 à 60% en poids, de préférence de 2,5 à 40% en poids, encore mieux de 2,7 à 20% en poids, voire de 2,75 à 15% en poids, par rapport au poids total de la composition.

### Alkylpolyglycosides

La composition selon l'invention comprend également un ou plusieurs tensioactifs non ioniques de type alkylpolyglycoside.
Ces tensioactifs peuvent être représentés par la formule générale suivante :

R₁O-(R₂O)ₜ-(G)ᵥ

dans laquelle:
- R₁ représente un radical alkyle saturé ou insaturé, linéaire ou ramifié comportant 6 à 24 atomes de carbone, notamment 8 à 18 atomes de carbone,
- R₂ représente un radical alkylène comportant 2 à 4 atomes de carbone, t désigne une valeur allant de 0 à 3, de préférence égale à 0, G désigne le glucose, le fructose ou le galactose, de préférence le glucose; le degré de polymérisation, c'est-à-dire la valeur de v, pouvant aller de 1 à 15, de préférence de 1 à 4; le degré moyen de polymérisation étant plus particulièrement compris entre 1 et 2.

Les liaisons glucosidiques entre les motifs sucre sont généralement de type 1-6 ou 1-4, de préférence de type 1-4.
De préférence, le tensioactif alkylpolyglycoside est un tensioactif alkylpolygluco-side.

Parmi les produits commerciaux, on peut citer les produits vendus par la société COGNIS sous les dénominations PLANTAREN® (600 CS/U, 1200 et 2000) ou PLANTACARE® (818, 1200 et 2000); les produits vendus par la société SEPPIC sous les dénominations TRITON CG110 (ou ORAMIX CG 10) et TRITON CG312 (ou ORAMIX® NS 10); les produits vendus par la société BASF sous la dénomination LUTENSOL GD 70 ou encore les produits vendus par la société CHEM Y sous la dénomination AG10 LK.
De préférence, on utilise les alkyl C8/C16-polyglycoside 1,4, notamment en solution aqueuse à 53%, tels que ceux commercialisés par COGNIS sous la référence PLANTACARE® 818 UP.

La composition selon l'invention comprend de préférence le ou lesdits tensioactifs non ioniques alkylpolyglycoside en une quantité inférieure ou égale à 10% en poids, notamment allant de 0,1 à 10% en poids, de préférence de 1 à 8% en poids, préférentiellement de 1,5 à 7,5% en poids, par rapport au poids total de la composition.

### Tensioactifs additionnels

La composition selon l'invention peut comprendre en outre un ou plusieurs tensioactifs additionnels, de préférence choisis parmi les tensioactifs non ioniques autres que les alkylpolyglycosides ci-dessus, les tensioactifs anioniques et les tensioactifs amphotères.

On entend par "tensioactif anionique", un tensioactif ne comportant à titre de groupements ioniques ou ionisables que des groupements anioniques. Ces groupements anioniques sont choisis de préférence parmi les groupements -C(O)OH, -C(O)O-, -SO3H, -S(O)2O-, -OS(O)2OH, -OS(O)2O-, -P(O)OH2, - P(O)2O-, -P(O)O2-, -P(OH)2, =P(O)OH, -P(OH)O-, =P(O)O-, =POH, =PO-, les parties anioniques comprenant un contre ion cationique tel que un métal alcalin, un métal alcalino-terreux, ou un ammonium.
Comme exemple de tensioactifs anioniques utilisables dans la composition selon l'invention, on peut citer les alkyl sulfates, les alkyl éther sulfates, les alkylamidoé-thersulfates, les alkylarylpolyéthersulfates, les monoglycéride-sulfates, les alkylsulfonates, les alkylamidesulfonates, les alkylarylsulfonates, les alpha-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates, les acylglutamates, les alkylsulfosuccinamates, les acyliséthionates et les N-acyltaurates, les sels de monoesters d'alkyle et d'acides polyglycoside-polycarboxyliques, les acyllactylates, les N-acyl glycinates, les sels d'acides D-galactoside-uroniques, les sels d'acides alkyl éther-carboxyliques, les sels d'acides alkyl aryl éther-carboxyliques, les sels d'acides alkyl amidoéthercarboxyliques, et les formes non salifiées correspondantes de tous ces composés, les groupes alkyle et acyle de tous ces composés comportant de 6 à 40 atomes de carbone et le groupe aryle désignant un groupe phényle. Ces composés peuvent être oxyéthylénés et comportent alors de préférence de 1 à 50 motifs oxyde d'éthylène.
Les sels de monoesters d'alkyle en C6-C24 et d'acides polyglycoside-polycarboxyliques peuvent être choisis parmi les polyglycoside-citrates d'alkyle en C6-C24, les polyglycosides-tartrates d'alkyle en C6-C24 et les polyglycoside-sulfosuccinates d'alkyle en C6-C24.
Lorsque les tensioactifs anioniques sont sous forme de sel, ils peuvent être choisis parmi les sels de métaux alcalins tels que le sel de sodium ou de potassium et de préférence de sodium, les sels d'ammonium, les sels d'amines et en particulier d'aminoalcools ou les sels de métaux alcalino-terreux tel que les sels de magnésium.
Come exemple de sels d'aminoalcools, on peut citer les sels de mono-, di- et triéthanolamine, les sels de mono-, di- ou triisopropanolamine, les sels de 2-amino-2-méthyl-1-propanol, 2-amino-2-méthyl-1,3-propanediol et tris(hydroxy-méthyl)amino méthane. On utilise de préférence les sels de métaux alcalins ou alcalino-terreux et en particulier les sels de sodium ou de magnésium.
Parmi les tensioactifs anioniques, on préfère tout particulièrement les alkyl(C6-C24)sulfates, les alkyl(C6-C24)éthersulfates comprenant de 2 à 50 motifs oxyde d'éthylène, notamment sous forme de sels de métaux alcalins, d'ammonium, d'aminoalcools, et de métaux alcalino-terreux, ou un mélange de ces composés.
Encore mieux, on préfère les alkyl(C12-C20)sulfates, les alkyl(C12-C20)éthersulfates comprenant de 2 à 20 motifs oxyde d'éthylène, notamment sous forme de sels de métaux alcalins, d'ammonium, d'aminoalcools, et de métaux alcalino-terreux, ou un mélange de ces composés. Mieux encore, on préfère le lauryl éther sulfate de sodium à 2,2 moles d'oxyde d'éthylène.

Les tensioactifs non ioniques additionnels susceptibles d'être utilisés peuvent être choisis parmi les alcools, les alpha-diols, les alkyl(C₁₋₂₀)phénols, ces composés étant polyéthoxylés, polypropoxylés ou ayant une chaîne grasse comportant, par exemple, de 8 à 30 atomes de carbone, notamment de 16 à 30 atomes de carbone, le nombre de groupements oxyde d'éthylène et/ou oxyde de propylène pouvant aller notamment de 2 à 50 et le nombre de groupements glycérol pouvant aller notamment de 2 à 30.
On peut également citer les condensats d'oxyde d'éthylène et d'oxyde de propylène sur des alcools gras; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4; les esters d'acides gras du sorbitane éthoxylés ayant de préférence de 2 à 40 motifs d'oxyde d'éthylène, les esters d'acides gras du saccharose, les esters d'acides gras du polyéthylèneglycol, les dérivés de N-(alkyl en C6-24)glucamine, les oxydes d'aminés tels que les oxydes d'(alkyl en C10-14)amines ou les oxydes de N-(acyl en C10-14)-aminopropylmorpholine.
Préférentiellement, on utilise les esters d'acides gras du sorbitane éthoxylés, les alcools gras polyéthoxylés et leurs mélanges.

Les tensioactifs amphotères additionnels susceptibles d'être utilisés dans l'invention peuvent être des dérivés d'amines aliphatiques secondaire ou tertiaire, éventuellement quaternisées, dans lesquels le groupe aliphatique est une chaîne linéaire ou ramifiée comportant 8 à 22 atomes de carbone, lesdits dérivés d'amines contenant au moins un groupe anionique tel que, par exemple, un groupe carboxylate, sulfonate, sulfate, phosphate ou phosphonate.
On peut citer en particulier les alkyl(C8-C20)bétaïnes, les sulfobétaïnes, les alkyl(C₈-C₂₀)sulfobétaïnes, les alkyl(C8-C20)amidoalkyl(C1-C6)bétaïnes telles que la cocoamidopropylbétaïne, les alkyl(C8-C20)amidoalkyl(C1-C6)sulfobétaïnes.

Parmi les dérivés d'amines aliphatiques secondaires ou tertiaires éventuellement quaternisées susceptibles d'être employés, on peut également citer les produits de structures respectives (A2) et (A3) suivantes :

(A2) Rₐ-CON(Z)CH₂-(CH₂)ₘ-N⁺(R_{b})(R_{c})(CH₂COO⁻)

dans laquelle :
Rₐ représente un groupe alkyle ou alkényle en C₁₀-C₃₀ dérivé d'un acide Rₐ-COOH de préférence présent dans l'huile de coprah hydrolysée, un groupe heptyle, nonyle ou undécyle,
R_{b} représente un groupe beta-hydroxyéthyle,
R_{c} représente un groupe carboxyméthyle ;
m est égal à 0,1 ou 2,
Z représente un atome d'hydrogène ou un groupe hydroxyéthyl ou carboxyméthyl;

(A3) R_{a'}-CON(Z)CH₂-(CH₂)_{m'}-N(B)(B')

dans laquelle :
B représente -CH₂CH₂OX', avec X' représentant -CH₂-COOH, CH₂-COOZ', - CH₂CH₂-COOH, -CH₂CH₂-COOZ', ou un atome d'hydrogène,
B' représente -(CH₂)_{z}-Y', avec z = 1 ou 2, et Y' représentant -COOH, -COOZ', - CH₂-CHOH-SO₃H ou -CH₂-CHOH-SO₃Z',
m' est égal à 0,1 ou 2,
Z représente un atome d'hydrogène ou un groupe hydroxyéthyl ou carboxyméthyl,
Z' représente un ion issu d'un métal alcalin ou alcalinoterreux, tel que le sodium, le potassium ou le magnésium; un ion ammonium; ou un ion issu d'une amine organique et notamment d'un aminoalcool, tel que la mono-, di- et triéthanolamine, la mono-, di- ou tri-isopropanol-amine, le 2-amino 2-méthyl 1-propanol, le 2-amino 2-méthyl 1,3-propanediol et le tris(hydroxyméthyl)amino méthane.
R_{a'} représente un groupe alkyle ou alkényle en C₁₀-C₃₀ d'un acide R_{a'}COOH de préférence présent dans l'huile de coprah ou dans l'huile de lin hydrolysée, un groupe alkyle, notamment en C₁₇ et sa forme iso, un groupe en C₁₇ insaturé.

Les composés répondant à la formule (A3) sont préférés. Ces composés sont également classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, co-coamphodipropionate de disodium, lauroamphodipropionate de disodium, caprylamphodipropionate de disodium, capryloamphodipropionate de disodium, acide lauroamphodipropionique, acide cocoamphodipropionique.
A titre d'exemple, on peut citer le cocoamphodiacétate commercialisé par la société RHODIA sous la dénomination commerciale MIRANOL® C2M concentré ou sous la dénomination commerciale MIRANOL ULTRA C 32 et le produit commercialisé par la société CHIMEX sous la dénomination commerciale CHIMEXANE HA.

On peut aussi utiliser des composés de formule (A4) :

(A4) R_{a"}-NH-CH(Y")-(CH₂)n-C(O)-NH-(CH₂)n'-N(R_{d})(Rₑ)

dans laquelle :
- R_{a"} représente un groupe alkyle ou alcényle en C₁₀-C₃₀ d'un acide R_{a"}-C(O)OH, de préférence présent dans l'huile de coprah ou dans l'huile de lin hydrolysée;
- Y" représente le groupe -C(O)OH, -C(O)OZ", -CH₂-CH(OH)-SO₃H ou le groupe
- CH₂-CH(OH)-SO₃-Z" avec Z" représentant un contre ion cationique issu d'un métal alcalin ou alcalinoterreux, tel que le sodium, un ion ammonium ou un ion issu d'une amine organique;
- R_{d} et Rₑ, indépendamment l'un de l'autre, représentent un radical alkyle ou hydroxyalkyle en C1-C4; et
- n et n', indépendamment l'un de l'autre, désignent un nombre entier allant de 1 à 3.
On peut notamment citer le composé classé dans le dictionnaire CTFA sous la dénomination sodium diethylaminopropyl cocoaspartamide et commercialisé par la société CHIMEX sous l'appellation CHIMEXANE HB.

De préférence, les tensioactifs amphotères sont choisis parmi les alkyl(C8-C20)bétaïnes, les alkyl(C8-C20)amidoalkyl(C1-C6)bétaïnes et les alkyl(C8-C20) amphodiacétates, ainsi que le sel de sodium du laurylaminosuccinamate de dié-thylaminopropyle; et leurs mélanges.

De préférence, la composition selon l'invention comprend une quantité totale de tensioactifs non ioniques (totaux c'est-à-dire alkylpolyglycosides et additionnels éventuels), anioniques (éventuels) et amphotères (éventuels), allant de 0,1 à 10% en poids, de préférence allant de 1 à 8% en poids, préférentiellement de 1,5 à 7,5% en poids, par rapport au poids total de la composition.
De préférence, la composition selon l'invention comprend une quantité totale de tensioactifs (cationiques, anioniques, non ioniques, amphotères, zwittérioniques) allant de 0,1 à 10% en poids, de préférence allant de 1 à 8% en poids, préférentiellement de 1,5 à 7,5% en poids, par rapport au poids total de la composition.

De préférence, le ratio (% en poids) "tensioactifs non ioniques (totaux) + tensioactifs anioniques (éventuels) + tensioactifs amphotères (éventuels)" / "polymères associatifs non ioniques" est inférieur ou égal à 3; de préférence il varie de 0,01 à 3, notamment de 0,01 à 2,8, préférentiellement de 0,1 à 2,5.

### Polymères

La composition selon l'invention peut comprendre en outre un ou plusieurs polymères, notamment choisis parmi les polymères amphotères ou cationiques, ainsi que leur mélange.

On entend par "polymère cationique", tout polymère comprenant des groupements cationiques et/ou des groupements ionisables en groupements cationiques. De préférence, le polymère cationique est hydrophile ou amphiphile. Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.
Les polymères cationiques susceptibles d'être utilisés ont de préférence une masse molaire moyenne en poids (Mw) comprise entre 500 et 5.10⁶ environ, de préférence comprise entre 10³ et 3.10⁶ environ.

Parmi les polymères cationiques, on peut citer plus particulièrement :
(1) les homopolymères ou copolymères dérivés d'esters ou d'amides acryliques ou méthacryliques et comportant au moins un des motifs de formule suivante : 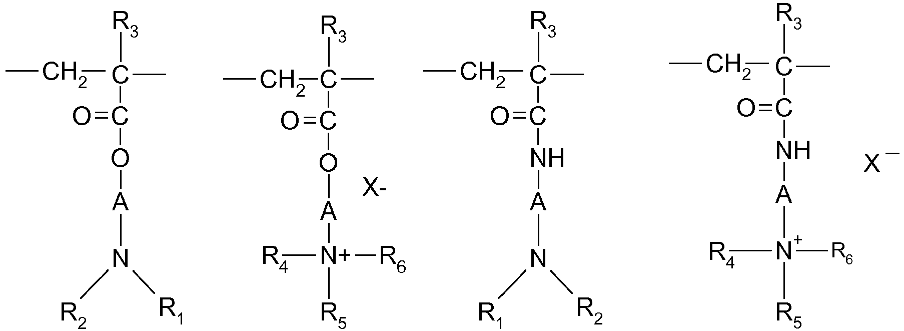 dans lesquelles:
   - R3, identiques ou différents, désignent un atome d'hydrogène ou un radical CH3;
   - A, identiques ou différents, représentent un groupe divalent alkyle, linéaire ou ramifié, de 1 à 6 atomes de carbone, de préférence 2 ou 3 atomes de carbone ou un groupe hydroxyalkyle de 1 à 4 atomes de carbone ;
   - R4, R5, R6, identiques ou différents, représentent un groupe alkyle ayant de 1 à 18 atomes de carbone ou un radical benzyle; de préférence un groupe alkyle ayant de 1 à 6 atomes de carbone;
   - R1 et R2, identiques ou différents, représentent un atome d'hydrogène ou un groupe alkyle ayant de 1 à 6 atomes de carbone, de préférence méthyle ou éthyle;
   - X désigne un anion dérivé d'un acide minéral ou organique tel qu'un anion méthosulfate ou un halogénure tel que chlorure ou bromure.
   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Parmi ces copolymères de la famille (1), on peut citer :
   - les copolymères d'acrylamide et de diméthylaminoéthyl méthacrylate quaternisé au sulfate de diméthyle ou avec un halogénure de diméthyle, tels que celui vendu sous la dénomination HERCOFLOC par la société HERCULES,
   - les copolymères d'acrylamide et de chlorure de méthacryloyloxyéthyltriméthylammonium, tels que ceux vendus sous la dénomination BINA QUAT P 100 par la société CIBA GEIGY,
   - le copolymère d'acrylamide et de méthosulfate de méthacryloyloxyéthyltriméthylammonium, tel que celui vendu sous la dénomination RETEN par la société HERCULES,
   - les copolymères vinylpyrrolidone/acrylate ou méthacrylate de dialkylaminoalkyle, quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthylaminoéthyle/ vinylcaprolactame/ vinylpyrrolidone, tel que le produit vendu sous la dénomination GAFFIX VC 713 par la société ISP,
   - les copolymères vinylpyrrolidone/ méthacrylamidopropyldimethylamine, tels que ceux commercialisés sous la dénomination STYLEZE CC 10 par ISP;
   - les copolymères vinylpyrrolidone/ méthacrylamide de diméthylaminopropyle quaternisé, tel que le produit vendu sous la dénomination "GAFQUAT HS 100" par la société ISP,
   - les polymères, de préférence réticulés, de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo- ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50% en poids dudit copolymère dans de l'huile minérale. Cette dispersion est commercialisée sous le nom de "SALCARE® SC 92" par la société CIBA. On peut également utiliser un homopolymère réticulé du chlorure de méthacryloyloxyéthyl triméthylammonium comprenant environ 50% en poids de l'homopolymère dans de l'huile minérale ou dans un ester liquide. Ces dispersions sont commercialisées sous les noms "SALCARE® SC 95" et "SALCARE® SC 96" par la société CIBA.
(2) Les polysaccharides cationiques, notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires sont notamment décrits dans FR1492597, et on peut citer les polymères commercialisés sous la dénomination "UCARE POLYMER JR" (JR 400 LT, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US4131576, et on peut citer les hydroxyalkylcelluloses, comme les hydroxyméthyl-, hydroxyéthyl- ou hydroxypropyl celluloses greffées notamment avec un sel de méthacryloyléthyl triméthylammonium, de méthacrylamidopropyl triméthylammonium, de diméthyl-diallylammonium. Les produits commercialisés répondant à cette définition sont plus particulièrement les produits vendus sous la dénomination "Celquat L 200" et "Celquat H 100" par la Société National Starch.
   Les gommes de galactomannane cationiques sont décrites plus particulièrement dans les brevets US3589578 et US4031307, et on peut citer les gommes de guar comprenant des groupements cationiques trialkylammonium. On utilise par exemple des gommes de guar modifiées par un sel (par exemple un chlorure) de 2,3-époxypropyl triméthylammonium. De tels produits sont commercialisés notamment sous les dénominations JAGUAR C13 S, JAGUAR C 15, JAGUAR C 17 ou JAGUAR C162 par la société RHODIA.
(3) les polymères constitués de motifs pipérazinyle et de radicaux divalents alkylène ou hydroxyalkylène à chaînes linéaires ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères.
(4) les polyaminoamides solubles dans l'eau, préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé; l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés.
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone; le rapport molaire entre le polyalkylène polyamine et l'acide dicarboxylique étant de préférence compris entre 0,8:1 et 1,4:1; le polyaminoamide en résultant étant amené à réagir avec l'épichlorhydrine dans un rapport molaire d'épichlorhydrine par rapport au groupement amine secondaire du polyaminoamide compris de préférence entre 0,5:1 et 1,8:1. Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (I) ou (II) : 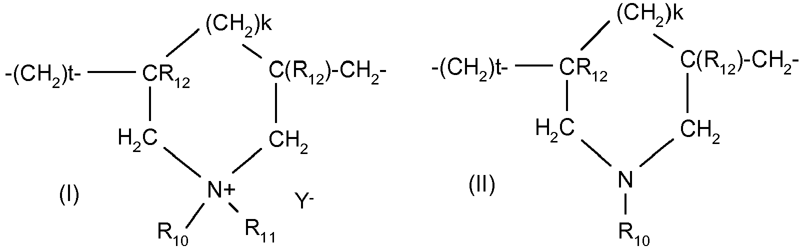 dans lesquelles
   - k et t sont égaux à 0 ou 1, la somme k + t étant égale à 1 ;
   - R12 désigne un atome d'hydrogène ou un radical méthyle ;
   - R10 et R11, indépendamment l'un de l'autre, désignent un groupement alkyle ayant de 1 à 6 atomes de carbone, un groupement hydroxyalkyle dans lequel le groupement alkyle a 1 à 5 atomes de carbone, un groupement amidoalkyle en C1-C4; ou bien R10 et R11 peuvent désigner conjointement avec l'atome d'azote auquel ils sont rattachés, des groupement hétérocycliques, tels que pipéridinyle ou morpholinyle ; R10 et R11, indépendamment l'un de l'autre, désignent de préférence un groupement alkyle ayant de 1 à 4 atomes de carbone.
   - Y⁻ est un anion tel que bromure, chlorure, acétate, borate, citrate, tartrate, bisulfate, bisulfite, sulfate, phosphate.
   On peut citer plus particulièrement l'homopolymère de sels (par exemple chlorure) de diméthyldiallylammonium par exemple vendu sous la dénomination "MERQUAT 100" par la société NALCO (et leurs homologues de faibles masses molaires moyenne en poids) et les copolymères de sels (par exemple chlorure) de diallyldiméthylammonium et d'acrylamide commercialisés notamment sous la dénomination "MERQUAT 550" ou "MERQUAT 7SPR".
(8) les polymères de diammonium quaternaire comprenant des motifs récurrents de formule : 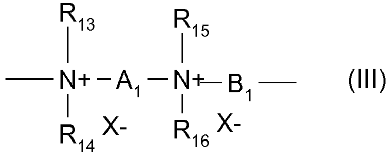 dans laquelle :
   - R13, R14, R15 et R16, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles comprenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
   - A1 et B1 représentent des groupements divalents polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   - X⁻ désigne un anion dérivé d'un acide minéral ou organique;
   Etant entendu que A1, R13 et R15 peuvent former avec les deux atomes d'azote auxquels ils sont rattachés un cycle pipérazinique ;
   en outre si A1 désigne un radical alkylène ou hydroxyalkylène linéaire ou ramifié, saturé ou insaturé, B1 peut également désigner un groupement (CH2)n-CO-D-OC-(CH2)n- dans lequel D désigne :
   a) un reste de glycol de formule -O-Z-O-, où Z désigne un radical hydrocarboné linéaire ou ramifié ou un groupement répondant à l'une des formules suivantes: -(CH2-CH2-O)x-CH2-CH2- et -[CH2-CH(CH3)-O]y-CH2-CH(CH3)- où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical divalent -CH2-CH2-S-S-CH2-CH2- ;
   d) un groupement uréylène de formule : -NH-CO-NH- ;
   De préférence, X⁻ est un anion tel que le chlorure ou le bromure. Ces polymères ont une masse molaire moyenne en nombre (Mn) généralement comprise entre 1000 et 100000.
   On peut citer plus particulièrement les polymères qui sont constitués de motifs récurrents répondant à la formule : 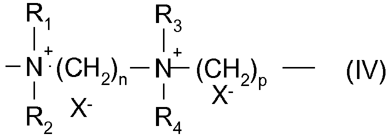 dans laquelle R1, R2, R3 et R4, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
   Un composé de formule (IV) particulièrement préféré est celui pour lequel R1, R2, R3 et R4 représentent un radical méthyle, n=3, p=6 et X = Cl, dénommé Hexadimethrine chloride selon la nomenclature INCI (CTFA).
(9) les polymères de polyammonium quaternaires comprenant des motifs de formule (V): 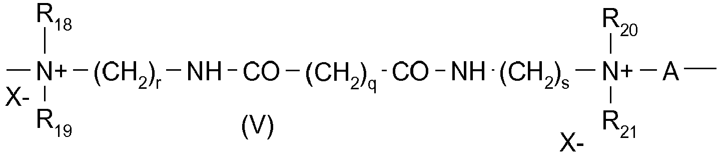 dans laquelle :
   - R18, R19, R20 et R21, identiques ou différents, représentent un atome d'hydrogène ou un radical méthyle, éthyle, propyle, β-hydroxyéthyle, β-hydroxypropyle ou
   - CH2CH2(OCH2CH2)pOH, où p est égal à 0 ou à un nombre entier compris entre 1 et 6, sous réserve que R18, R19, R20 et R21 ne représentent pas simultanément un atome d'hydrogène,
   - r et s, identiques ou différents, sont des nombres entiers compris entre 1 et 6,
   - q est égal à 0 ou à un nombre entier compris entre 1 et 34,
   - X- désigne un anion tel qu'un halogénure,
   - A désigne un radical d'un dihalogénure ou représente de préférence
   - CH2-CH2-O-CH2-CH2-.
   On peut par exemple citer les produits "Mirapol® A 15", "Mirapol® AD1", "Mirapol® AZ1" et "Mirapol® 175" vendus par la société Miranol.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par COGNIS, référencé sous le nom de "POLYETHYLENEGLYCOL (15) TALLOW POLYAMINE" dans le dictionnaire CTFA.
(12) les polymères comportant dans leur structure :
   (a) un ou plusieurs motifs répondant à la formule (A) suivante : 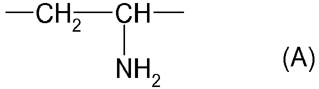
   (b) éventuellement un ou plusieurs motifs répondant à la formule (B) suivante :

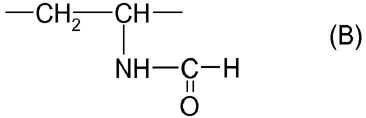

Autrement dit, ces polymères peuvent être notamment choisis parmi les homo- ou copolymères comportant un ou plusieurs motifs issus de la vinylamine et éventuellement un ou plusieurs motifs issus du vinylformamide.

De préférence, ces polymères cationiques sont choisis parmi les polymères comportant, dans leur structure, de 5 à 100% en moles de motifs répondant à la formule (A) et de 0 à 95% en moles de motifs répondant à la formule (B), préférentiellement de 10 à 100% en moles de motifs répondant à la formule (A) et de 0 à 90% en moles de motifs répondant à la formule (B).
Ces polymères peuvent être obtenus par exemple par hydrolyse partielle du polyvinylformamide. Cette hydrolyse peut se faire en milieu acide ou basique.
La masse moléculaire moyenne en poids dudit polymère, mesurée par diffraction de la lumière, peut varier de 1000 à 3.000.000 g/mole, de préférence de 10 000 à 1.000.000 et plus particulièrement de 100 000 à 500.000 g/mole.
La densité de charge cationique de ces polymères peut varier de 2 meq/g à 20 meq/g, de préférence de 2,5 à 15 et plus particulièrement de 3,5 à 10 meq/g.
Les polymères comportant des motifs de formule (A) et éventuellement des motifs de formule (B) sont notamment vendus sous la dénomination LUPAMIN par la société BASF, tels que par exemple, et de manière non limitative, les produits proposés sous la dénomination LUPAMIN 9095, LUPAMIN 5095, LUPAMIN 1095, LUPAMIN 9030 (ou LUVIQUAT 9030) et LUPAMIN 9010.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

De préférence, les polymères cationiques sont choisis parmi ceux des familles (1), (2), (7) et (10) ci-dessus citées.
Parmi les polymères cationiques mentionnés ci-dessus, on peut utiliser de préférence les polysaccharides cationiques, notamment les celluloses et les gommes de galactomannanes cationiques, et en particulier les dérivés d'éther de cellulose quaternaires tels que les produits vendus sous la dénomination "JR 400" par la Société AMERCHOL, les cyclopolymères cationiques, en particulier les homopolymères ou copolymères de sels (par exemple chlorure) de diméthyldiallylammonium, vendus sous les dénominations MERQUAT 100, MERQUAT 550 et MERQUAT S par la société NALCO et leurs homologues de faibles poids moléculaires en poids, les polymères quaternaires de vinylpyrrolidone et de vinylimidazole, les homopolymères ou copolymères éventuellement réticulés de sels de méthacryloyloxyalkyl(C1-C4) trialkyl(C1-C4)ammonium; et leurs mélanges.

Il est également possible d'utiliser des polymères amphotères, qui peuvent de préférence être choisis parmi les polymères amphotères comprenant la répétition de :
(i) un ou plusieurs motifs issus d'un monomère de type (méth)acrylamide,
(ii) un ou plusieurs motifs issus d'un monomère de type (méth)acrylamidoalkyltrialkylammonium, et
(iii) un ou plusieurs motifs issus d'un monomère acide de type acide (méth)acrylique.

De préférence, les motifs issus d'un monomère de type (i) (méth)acrylamide sont des motifs de structure (la) suivante : 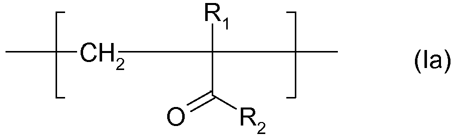 dans laquelle R₁ désigne H ou CH₃, et R₂ est choisi parmi un radical amino, diméthylamino, tert-butylamino, dodécylamino, ou -NH-CH₂OH.
De préférence, ledit polymère amphotère ne comprend la répétition que d'un seul motif de formule (la).
Le motif issu d'un monomère de type (méth)acrylamide de formule (la) dans laquelle R₁ désigne H et R₂ est un radical amino (NH2) est particulièrement préféré. Il correspond au monomère acrylamide proprement dit.

De préférence, les motifs issus d'un monomère de type (ii) (méth)acrylamidoalkyltrialkylammonium sont des motifs de structure (IIa) : 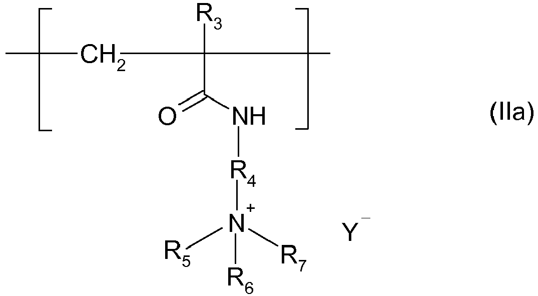 dans laquelle :
- R₃ désigne H ou CH₃,
- R₄ désigne un groupement (CH₂)k avec k un nombre entier allant de 1 à 6, et de préférence de 2 à 4 ;
- R₅, R₆ et R₇, identiques ou différents, désignent chacun un groupement alkyle ayant de 1 à 4 atomes de carbone;
- Y⁻ est un anion tel que bromure, chlorure, acétate, borate, citrate, tartrate, bisulfate, bisulfite, sulfate, phosphate.
De préférence, ledit polymère amphotère ne comprend la répétition que d'un seul motif de formule (IIa).
Parmi ces motifs issus d'un monomère de type (méth)acrylamidoalkyltrialkylammonium de formule (IIa), on préfère ceux issus du monomère chlorure de méthacrylamidopropyltriméthylammonium, pour lequel R₃ désigne un radical méthyle, k vaut 3, R₅, R₆ et R₇ désignent un radical méthyle, et Y⁻ désigne un anion chlorure.

De préférence, les motifs issus d'un monomère de type (iii) acide (méth)acrylique sont des motifs de formule (IIIa) : 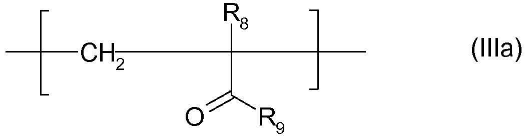 dans laquelle R₈ désigne H ou CH₃, et R₉ désigne un radical hydroxyle ou un radical -NH-C(CH₃)₂-CH₂-SO₃H.
Les motifs préférés de formules (IIIa) correspondent aux monomères acide acrylique, acide méthacrylique et acide 2-acrylamino 2-méthyl propane sulfonique.
De préférence, le motif issu d'un monomère de type acide (méth)acrylique de formule (IIIa) est celui issu de l'acide acrylique, pour lequel R₈ désigne un atome d'hydrogène et R₉ désigne un radical hydroxyle.
Le ou les monomères acides de type acide (méth)acrylique peuvent être non neutralisés, ou partiellement ou totalement neutralisés par une base organique ou minérale.
De préférence, ledit polymère amphotère ne comprend la répétition que d'un seul motif de formule (IIIa).

Selon un mode de réalisation préféré de l'invention, le ou les polymères amphotères de ce type comprennent au moins 30% en mole de motifs issus d'un monomère de type (i) (méth)acrylamide. De préférence, ils comprennent de 30 à 70% en mole de motifs issus d'un monomère de type (méth)acrylamide, de manière plus préférée de 40 à 60% en mole.
La teneur en motifs issus d'un monomère de type (ii) (méth)acrylamido alkyltrialkylammonium peut avantageusement être de 10 à 60%, préférentiellement de 20 à 55% en moles.
La teneur en motifs issus d'un monomère acide de type (iii) acide (méth)acrylique peut avantageusement être de 1 à 20%, préférentiellement de 5 à 15% en moles. Selon un mode de réalisation particulièrement préféré de l'invention, le polymère amphotère de ce type comprend :
- de 30 à 70% en moles de motifs issus d'un monomère de type (i) (méth)acrylamide, de manière plus préférée de 40 à 60% en moles,
- de 10 à 60% en moles, préférentiellement de 20 à 55% en moles, de motifs issus d'un monomère de type (ii) (méth)acrylamidoalkyltrialkylammonium, et
- de 1 à 20% en moles, préférentiellement de 5 à 15% en moles, de motifs issus d'un monomère de type (iii) acide (méth)acrylique.

Ce type de polymères amphotères peut également comprendre des motifs additionnels, différents des motifs issus d'un monomère de type (méth)acrylamide, de type (méth)acrylamidoalkyltrialkylammonium et de type acide (méth)acrylique tels que décrits ci-avant.
Toutefois, selon un mode de réalisation préféré de l'invention, lesdits polymères amphotères sont constitués uniquement de motifs issus de monomères de type (i)
(méth)acrylamide, de type (ii) (méth)acrylamidoalkyltrialkylammonium et de type (iii) acide (méth)acrylique.
Comme exemple de polymères amphotères particulièrement préférés, on peut citer les terpolymères acrylamide/chlorure de méthacrylamidopropyltriméthylammonium/acide acrylique. De tels polymères sont répertoriés dans le dictionnaire C.T.F.A. International Cosmetic Ingredient Dictionary, 10ème édition 2004, sous la dénomination "Polyquaternium 53". Des produits correspondants sont notamment commercialisés sous les dénominations MERQUAT 2003 et MERQUAT 2003 PR par la société NALCO.

Comme autre type de polymère amphotère susceptible d'être utilisé, on peut également citer les copolymères à base d'acide (méth)acrylique et d'un sel de dialkyldiallylammonium, tels que les copolymères d'acide (méth)acrylique et de chlorure de diméthyldiallyl ammonium. On peut citer par exemple le Merquat 280 proposé par la société NALCO.

La composition selon l'invention peut comprendre les polymères cationiques et/ou amphotères en une quantité comprise entre 0,01 et 5% en poids, notamment de 0,05 à 3% en poids, préférentiellement de 0,1 à 2% en poids, par rapport au poids total de la composition.

### Autres ingrédients

La composition cosmétique selon l'invention peut se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, d'un gel aqueux ou anhydre, ou de toute autre forme cosmétique.

La composition selon l'invention est de préférence aqueuse et comprend alors de l'eau à une concentration allant de préférence de 5 à 98% en poids, notamment de 20 à 95% en poids, mieux de 50 à 90% en poids, par rapport au poids total de la composition.
La composition peut également comprendre un ou plusieurs solvants organiques liquides à 25°C, 1 atm., notamment hydrosolubles, tels que les alcools en C1-C7, et notamment les monoalcools aliphatiques ou aromatiques en C1-C7, les polyols et les éthers de polyols en C3-C7, qui peuvent donc être employés seuls ou en mélange avec de l'eau. Avantageusement, le solvant organique peut être choisi parmi l'éthanol, l'isopropanol et leurs mélanges.

La composition selon l'invention peut en outre comprendre au moins un ingrédient cosmétique usuel, différent des composés de l'invention, et notamment choisi parmi les huiles végétales, minérales, animales ou de synthèse; les corps gras solides et notamment les cires, les esters en C8-C40, les acides en C8-C40; les alcools en C8-C40; les tensioactifs cationiques, les polymères anioniques; les filtres solaires; les agents hydratants; les agents antipelliculaires; les agents antioxydants; les agents chélatants; les agents nacrants et opacifiants; les agents plastifiants ou de coalescence; les hydroxyacides; les charges; les silicones et en particulier les polydiméthylsiloxanes (PDMS); les parfums; les agents d'alcalinisation ou d'acidification; les aldéhydes, la DHA; les épaississants autres que les polymères associatifs non ioniques selon l'invention, et de préférence choisis parmi les épaississants non polymériques et les épaississants polymériques non associatifs. La composition peut bien évidemment comprendre plusieurs ingrédients cosmétiques figurant dans la liste ci-dessus. L'homme de métier veillera à choisir les ingrédients entrant dans la composition, ainsi que leurs quantités, de manière à ce qu'ils ne nuisent pas aux propriétés des compositions de la présente invention.

Le pH de la composition, si elle est aqueuse, est de préférence compris entre 3 et 9, notamment entre 3 et 6.

La composition cosmétique selon l'invention trouve notamment une application particulièrement intéressante dans le domaine du soin et de l'hygiène capillaire, notamment pour le soin et/ou le nettoyage des cheveux et/ou du cuir chevelu.
La composition cosmétique peut être rincée ou non rincée après avoir été appliquée sur les cheveux et/ou le cuir chevelu; de préférence elle est rincée, après un éventuel temps de pose.
La composition selon l'invention peut être conditionnée dans un tube, dans un flacon muni ou non d'une pompe, ou encore dans un aérosol. Dans le cas d'un aérosol, la composition peut alors contenir un ou plusieurs agents propulseurs classiques.

L'invention a également pour objet un procédé de traitement cosmétique, notamment de soin et/ou de nettoyage, des cheveux et/ou du cuir chevelu, comprenant l'application sur les cheveux et/ou le cuir chevelu, d'une composition cosmétique selon l'invention, suivie éventuellement d'un rinçage, après un éventuel temps de pose.

L'invention est illustrée plus en détails dans les exemples suivants.

### Exemples 1 à 18

On prépare les compositions capillaires suivantes (% en poids de matière commerciale) :

| | Exemple 1 | Exemple 2 | Exemple 3 | Exemple 4 |
|---|---|---|---|---|
| PEG-136/STEARETH-100/HDI COPOLYMER *(RHEOLATE FX1100)* | 3 | 3 | 3 | 3 |
| COCO-GLUCOSIDE à 52% (PLANTACARE 818 UP) | 12 | 9 | 9 | 9 |
| DISODIUM COCOYL GLUTAMATE à 31,3% *(PLANTAPON ACG LC)* | 2 | 5 | 5 | 5 |
| POLYQUATERNIUM-10 *(UCARE POLYMER JR 400 LT)* | 0,2% MA | 0,3% MA | | |
| POLYQUATERNIUM-53 (*MERQUAT 2003PR*) | | | 1% MA | |
| POLYQUATERNIUM-67 (*SOFTCAT POLYMER SL-100*) | | | | 0,2% MA |
| NaCl | | | 0,7 | |
| Eau | QSP 100 % | QSP 100 % | QSP 100 % | QSP 100 % |

| | Exemple 5 | Exemple 6 |
|---|---|---|
| PEG-136/STEARETH-100/HDI COPOLYMER *(RHEOLATE FX1100)* | 2,75 | 2,5 |
| Copolymère acrylate (*AQUA SF-1 de LUBRIZOL*) | 0,8% | 1,7% |
| COCO-GLUCOSIDE à 52% (PLANTACARE 818 UP) | 9 | 9 |
| DISODIUM COCOYL GLUTAMATE à 31,3% *(PLANTAPON ACG LC)* | 5 | 5 |
| Aminométhylpropanol | 0,085 | 0,17 |
| Eau | QSP 100 % | QSP 100 % |

| | Exemple 7 | Exemple 8 | Exemple 9 | Exemple 10 |
|---|---|---|---|---|
| PEG-136/STEARETH-100/HDI COPOLYMER *(RHEOLATE FX1100)* | 3 | 3 | 3 | 3 |
| COCO-GLUCOSIDE à 52% (PLANTACARE 818 UP) | 9 | 9 | 9 | 9 |
| DISODIUM COCOYL GLUTAMATE à 31,3% *(PLANTAPON ACG LC)* | | 5 | 5 | 5 |
| Vinylamine/VINYLFORMAMIDE COPOLYMER (*LUVIQUAT 9030*) | 7,5% MA | | | |
| POLYQUATERNIUM-10 *(UCARE POLYMER JR 400 LT)* | | 0,2% MA | 0,3% MA | 0,2% MA |
| 2-OLEAMIDO-1,3-OCTADECANEDIOL (*MEXANYL GZ CHIMEX*) | | | | 0,01 |
| Eau | QSP 100 % | QSP 100 % | QSP 100 % | QSP 100 % |

| | Exemple 11 | Exemple 12 | Exemple 13 | Exemple 14 |
|---|---|---|---|---|
| PEG-136/STEARETH-100/HDI COPOLYMER *(RHEOLATE FX1100)* | 3 | 3 | 3 | 3 |
| COCO-GLUCOSIDE à 52% (PLANTACARE 818 UP) | 9 | 9 | 9 | 9 |
| DISODIUM COCOYL GLUTAMATE à 31,3% *(PLANTAPON ACG LC)* | 5 | 5 | 5 | 5 |
| POLYQUATERNIUM-10 *(UCARE POLYMER JR 400 LT)* | 0,2% MA | | | |
| POLYQUATERNIUM-53 (*MERQUAT 2003PR POLYMER*) | | 1% MA | 1% MA | |
| NaCl | | 1 | 1 | |
| 2-OLEAMIDO-1,3-OCTADECANEDIOL (*MEXANYL GZ CHIMEX*) | 0,1 | 0,01 | 0,1 | |
| PIROCTONE OLAMINE *(OCTO-PYROX CLARIANT)* | | | | 0,5 |
| Eau | QSP 100 % | QSP 100 % | QSP 100 % | QSP 100 % |

| | Exemple 15 | Exemple 16 | Exemple 17 | Exemple 18 |
|---|---|---|---|---|
| PEG-136/STEARETH-100/HDI COPOLYMER *(RHEOLATE FX1100)* | 3 | 3 | 3 | 3 |
| COCO-GLUCOSIDE à 52% (PLANTACARE 818 UP) | 9 | 9 | 9 | 9 |
| CETRIMONIUM CHLORIDE (*DEHYQUART A OR COGNIS*) | 4 | | 4 | |
| BEHENTRIMONIUM CHLORIDE (*GENAMIN KDMP CLARIANT*) | | 1,2% MA | | 1,2% MA |
| CETEARYL ALCOHOL (*LA-NETTE O OR*) | | | 2 | 2 |
| Eau | QSP 100% | QSP 100% | QSP 100% | QSP 100% |

On obtient des compositions détergentes à effet moussant, susceptibles d'être employées pour le nettoyage des cheveux et conduisant à de bonnes propriétés cosmétiques sur cheveux secs.

## Revendications

1. Composition cosmétique non colorante comprenant :
(i) 2,5 à 60% en poids par rapport au poids total de la composition, d'un ou plusieurs polymères non ioniques associatifs, et
(ii) un ou plusieurs tensioactifs non ioniques de type alkylpolyglycoside de formule:
R₁O-(R₂O)ₜ-(G)ᵥ
dans laquelle:
- R₁ représente un radical alkyle saturé ou insaturé, linéaire ou ramifié comportant 6 à 24 atomes de carbone, notamment 8 à 18 atomes de carbone,
- R₂ représente un radical alkylène comportant 2 à 4 atomes de carbone,
- G désigne le glucose, le fructose ou le galactose, de préférence le glucose;
- t désigne une valeur allant de 0 à 3, de préférence égale à 0,
- v désigne une valeur allant de 1 à 15, de préférence de 1 à 4 ;
composition dans laquelle les polymères non ioniques associatifs sont des polyuréthanes polyéthers copolymères triblocs dont la séquence hydrophile est une chaîne polyoxyéthylénée comportant de 50 à 1000, notamment de 100 à 300, groupements oxyéthylénés; et comportant au moins deux chaînes lipophiles hydrocarbonées ayant de 6 à 30 atomes de carbone, séparées par ladite séquence hydrophile, lesdites chaînes lipophiles hydrocarbonées pouvant être des chaînes pendantes ou des chaînes en bout de séquence hydrophile.

2. Composition selon la revendication 1, dans laquelle les polymères non ioniques associatifs sont choisis parmi :
- les polyuréthanes polyéthers non ioniques associatifs susceptibles d'être obtenus par polycondensation d'au moins trois composés comprenant (i) au moins un polyéthylèneglycol comprenant de 100 à 180 moles d'oxyde d'éthylène, (ii) un alcool stéarylique polyoxyéthyléné comprenant 100 moles d'oxyde d'éthylène et (iii) un diisocyanate.
- les polyuréthanes polyéthers non ioniques associatifs susceptibles d'être obtenus par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

3. Composition selon l'une quelconque des revendications précédentes, comprenant les polymères non ioniques associatifs en une quantité allant de 2,5 à 40% en poids, encore mieux de 2,7 à 20% en poids, voire de 2,75 à 15% en poids, par rapport au poids total de la composition.

4. Composition selon l'une quelconque des revendications précédentes, comprenant les tensioactifs non ioniques alkylpolyglycoside en une quantité inférieure ou égale à 10% en poids, notamment allant de 0,1 à 10% en poids, de préférence de 1 à 8% en poids, préférentiellement de 1,5 à 7,5% en poids, par rapport au poids total de la composition.

5. Composition selon l'une quelconque des revendications précédentes, comprenant en outre un ou plusieurs tensioactifs additionnels choisis parmi les tensioactifs non ioniques autres que les alkylpolyglycosides, les tensioactifs anioniques et les tensioactifs amphotères.

6. Composition selon l'une quelconque des revendications précédentes, comprenant une quantité totale de tensioactifs non ioniques, anioniques et amphotères, allant de 0,1 à 10% en poids, de préférence allant de 1 à 8% en poids, préférentiellement de 1,5 à 7,5% en poids, par rapport au poids total de la composition.

7. Composition selon l'une quelconque des revendications précédentes, comprenant une quantité totale de tensioactifs allant de 0,1 à 10% en poids, de préférence allant de 1 à 8% en poids, préférentiellement de 1,5 à 7,5% en poids, par rapport au poids total de la composition.

8. Composition selon l'une quelconque des revendications précédentes, dans laquelle le ratio (% en poids) "tensioactifs non ioniques + tensioactifs anioniques + tensioactifs amphotères" / "polymères associatifs non ioniques" est inférieur ou égal à 3; de préférence varie de 0,01 à 3, notamment de 0,01 à 2,8, préférentiellement de 0,1 à 2,5.

9. Composition selon l'une quelconque des revendications précédentes, comprenant en outre un ou plusieurs polymères, notamment choisis parmi les polymères amphotères et cationiques, ainsi que leur mélange.

10. Composition selon l'une quelconque des revendications précédentes, comprenant de l'eau à une concentration allant de préférence de 5 à 98% en poids, notamment de 20 à 95% en poids, mieux de 50 à 80% en poids, par rapport au poids total de la composition.

11. Procédé de traitement cosmétique, notamment de soin et/ou de nettoyage, des cheveux et/ou du cuir chevelu, comprenant l'application sur les cheveux et/ou le cuir chevelu, d'une composition cosmétique telle que définie à l'une des revendications 1 à 10, suivie éventuellement d'un rinçage, après un éventuel temps de pose.

## Patentansprüche

1. Nicht färbende kosmetische Zusammensetzung, umfassend:
(i) 2,5 bis 60 Gew.-%, bezogen auf das Gesamtgewicht der Verbindung, eines oder mehrerer assoziativer nichtionischer Polymere und
(ii) ein oder mehrere nichtionische Tenside vom Alkylpolyglycosid-Typ der Formel:
R₁O-(R₂O)ₜ(G)ᵥ,
in der:
- R₁ für einen linearen oder verzweigten, gesättigten oder ungesättigten Alkylrest mit 6 bis 24 Kohlenstoffatomen, insbesondere 8 bis 18 Kohlenstoffatomen, steht,
- R₂ für einen Alkylenrest mit 2 bis 4 Kohlenstoffatomen steht,
- G für Glucose, Fructose oder Galactose, vorzugsweise Glucose, steht,
- t für einen Wert im Bereich von 0 bis 3 steht und vorzugsweise gleich 0 ist,
- v für einen Wert im Bereich von 1 bis 15, vorzugsweise von 1 bis 4, steht;
wobei es sich in der Zusammensetzung bei den assoziativen nichtionischen Polymeren um Triblock-Polyurethanpolyether-Copolymere handelt, in denen es sich bei dem hydrophilen Block um eine Polyoxyethylenkette mit 50 bis 1000 und speziell 100 bis 300 Oxyethylengruppen handelt und die mindestens zwei kohlenwasserstoffbasierte lipophile Ketten mit 6 bis 30 Kohlenstoffatomen, die durch den hydrophilen Block getrennt sind, umfassen, wobei es sich bei den kohlenwasserstoffbasierten lipophilen Ketten um Seitenketten oder Ketten am Ende eines hydrophilen Blocks handeln kann.

2. Zusammensetzung nach Anspruch 1, wobei die assoziativen nichtionischen Polymere aus
- assoziativen nichtionischen Polyurethanpolyethern, die durch Polykondensation von mindestens drei Verbindungen, umfassend (i) mindestens ein Polyethylenglykol mit 100 bis 180 mol Ethylenoxid, (ii) einen polyoxyethylenierten Stearylalkohol mit 100 mol Ethylenoxid und (iii) ein Diisocyanat, erhältlich sind;
- assoziativen nichtionischen Polyurethanpolyethern, die durch Polykondensation von mindestens drei Verbindungen, umfassend (i) mindestens ein Polyethylenglykol mit 100 bis 180 mol Ethylenoxid, (ii) Stearylalkohol oder Decylalkohol und (iii) mindestens ein Diisocyanat, erhältlich sind; ausgewählt sind.

3. Zusammensetzung nach einem der vorhergehenden Ansprüche, umfassend die assoziativen nichtionischen Polymere in einer Menge im Bereich von 2,5 bis 40 Gew.-%, noch besser 2,7 bis 20 Gew.-% oder sogar 2,75 bis 15 Gew.-%, bezogen auf das Gesamtgewicht der Zusammensetzung.

4. Zusammensetzung nach einem der vorhergehenden Ansprüche, umfassend die nichtionischen Alkylpolyglycosid-Tenside in einer Menge kleiner oder gleich 10 Gew.-%, insbesondere im Bereich von 0,1 bis 10 Gew.-%, vorzugsweise von 1 bis 8 Gew.-%, bevorzugt von 1,5 bis 7,5 Gew.-%, bezogen auf das Gesamtgewicht der Zusammensetzung.

5. Zusammensetzung nach einem der vorhergehenden Ansprüche, außerdem umfassend ein oder mehrere zusätzliche Tenside, die aus nichtionischen Tensiden, die von den Alkylpolyglycosiden verschieden sind, anionischen Tensiden und amphoteren Tensiden ausgewählt sind.

6. Zusammensetzung nach einem der vorhergehenden Ansprüche, umfassend eine Gesamtmenge von nichtionischen, anionischen und amphoteren Tensiden im Bereich von 0,1 bis 10 Gew.-%, vorzugsweise im Bereich von 1 bis 8 Gew.-% und bevorzugt von 1,5 bis 7,5 Gew.-%, bezogen auf das Gesamtgewicht der Zusammensetzung.

7. Zusammensetzung nach einem der vorhergehenden Ansprüche, umfassend eine Gesamtmenge von Tensiden im Bereich von 0,1 bis 10 Gew.-%, vorzugsweise im Bereich von 1 bis 8 Gew.-% und bevorzugt von 1,5 bis 7,5 Gew.-%, bezogen auf das Gesamtgewicht der Zusammensetzung.

8. Zusammensetzung nach einem der vorhergehenden Ansprüche, wobei das Verhältnis (Gew.-%) "nichtionische Tenside + anionische Tenside + amphotere Tenside"/"nichtionische assoziative Polymere" kleiner oder gleich 3 ist und vorzugsweise im Bereich von 0,01 bis 3, insbesondere von 0,01 bis 2,8 und bevorzugt von 0,1 bis 2,5 liegt.

9. Zusammensetzung nach einem der vorhergehenden Ansprüche, außerdem umfassend ein oder mehrere Polymere, die insbesondere aus amphoteren und kationischen Polymeren sowie Mischungen davon ausgewählt sind.

10. Zusammensetzung nach einem der vorhergehenden Ansprüche, umfassend Wasser in einer Konzentration, die vorzugsweise im Bereich von 5 bis 98 Gew.-%, insbesondere von 20 bis 95 Gew.-%, noch besser von 50 bis 80 Gew.-%, bezogen auf das Gesamtgewicht der Zusammensetzung, liegt.

11. Verfahren zu kosmetischen Behandlung, insbesondere zur Pflege und/oder Reinigung des Haars und/oder der Kopfhaut, bei dem man auf das Haar und/oder die Kopfhaut eine kosmetische Zusammensetzung gemäß einem der Ansprüche 1 bis 10 aufbringt und gegebenenfalls nach einer fakultativen Einwirkungszeit spült.

## Claims

1. Non-colouring cosmetic composition comprising:
(i) 2.5% to 60% by weight, relative to the total weight of the composition, of one or more associative nonionic polymers, and
(ii) one or more nonionic surfactants of the alkylpolyglycoside type of formula:
R₁O-(R₂O)ₜ-(G)ᵥ
in which:
- R₁ represents a linear or branched, saturated or unsaturated alkyl radical comprising 6 to 24 carbon atoms and especially 8 to 18 carbon atoms,
- R₂ represents an alkylene radical comprising 2 to 4 carbon atoms,
- G denotes glucose, fructose or galactose, preferably glucose;
- t denotes a value ranging from 0 to 3 and preferably equal to 0,
- v denotes a value ranging from 1 to 15 and preferably 1 to 4;
composition in which the associative nonionic polymers are triblock polyurethane polyether copolymers in which the hydrophilic block is a polyoxyethylene chain comprising from 50 to 1000 and especially from 100 to 300 oxyethylene groups; and comprising at least two hydrocarbon-based lipophilic chains containing from 6 to 30 carbon atoms, separated by the said hydrophilic block, the said hydrocarbon-based lipophilic chains possibly being pendent chains or chains at the end of a hydrophilic block.

2. Composition according to Claim 1, in which the associative nonionic polymers are chosen from:
- associative nonionic polyurethane polyethers that may be obtained by polycondensation of at least three compounds comprising (i) at least one polyethylene glycol comprising from 100 to 180 mol of ethylene oxide, (ii) a polyoxyethylenated stearyl alcohol comprising 100 mol of ethylene oxide, and (iii) a diisocyanate;
- associative nonionic polyurethane polyethers that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.

3. Composition according to either one of the preceding claims, comprising the associative nonionic polymers in an amount ranging from 2.5% to 40% by weight, better still from 2.7% to 20% by weight or even from 2.75% to 15% by weight, relative to the total weight of the composition.

4. Composition according to any one of the preceding claims, comprising the alkylpolyglycoside nonionic surfactants in an amount of less than or equal to 10% by weight, especially ranging from 0.1% to 10% by weight, preferably from 1% to 8% by weight and preferentially from 1.5% to 7.5% by weight, relative to the total weight of the composition.

5. Composition according to any one of the preceding claims, also comprising one or more additional surfactants chosen from nonionic surfactants other than the alkylpolyglycosides, anionic surfactants and amphoteric surfactants.

6. Composition according to any one of the preceding claims, comprising a total amount of nonionic, anionic and amphoteric surfactants ranging from 0.1% to 10% by weight, preferably ranging from 1% to 8% by weight and preferentially from 1.5% to 7.5% by weight, relative to the total weight of the composition.

7. Composition according to any one of the preceding claims, comprising a total amount of surfactants ranging from 0.1% to 10% by weight, preferably ranging from 1% to 8% by weight and preferentially from 1.5% to 7.5% by weight, relative to the total weight of the composition.

8. Composition according to any one of the preceding claims, in which the ratio (weight percentage) "nonionic surfactants + anionic surfactants + amphoteric surfactants"/"nonionic associative polymers" is less than or equal to 3; it preferably ranges from 0.01 to 3, especially from 0.01 to 2.8 and preferentially from 0.1 to 2.5.

9. Composition according to one of the preceding claims, also comprising one or more polymers, especially chosen from amphoteric and cationic polymers, and also mixtures thereof.

10. Composition according to any one of the preceding claims, comprising water at a concentration preferably ranging from 5% to 98% by weight, especially from 20% to 95% by weight and better still from 50% to 80% by weight, relative to the total weight of the composition.

11. Cosmetic treatment process, especially for caring for and/or cleansing the hair and/or the scalp, comprising the application to the hair and/or the scalp of a cosmetic composition as defined in one of Claims 1 to 10, optionally followed by rinsing, after an optional leave-on time.
